# EUROPEAN PATENT SPECIFICATION

(11) **EP 1 260 203 B1**
(45) Date of publication and mention of the grant of the patent: **28.09.2005**
(21) Application number: 02005323.7
(22) Date of filing: 13.03.2002
(51) Int. Cl.: A61F 13/15

(54) **Apparatus and method for manufacturing wearing articles**
Verfahren und Vorrichtung zur Herstellung von Kleidungsstücken
Procédé et dispositif de production de vêtements

(30) Priority: 23.05.2001 JP 2001153392; 21.12.2001 JP 2001388762
(43) Date of publication of application: 27.11.2002
(73) Proprietor: Zuiko Corporation, Osaka 566-0045 (JP)
(72) Inventor: Nakakado, Masaki, Osaka 573-0065 (JP); Satoh, Hitoshi, Osaka 563-0036 (JP); Yomeoka, Kikuo, Osaka 597-0091 (JP)
(74) Representative: Schwabe - Sandmair - Marx

(56) References cited:
- WO-A-96/23477
- US-A- 5 147 487

## Description

### FIELD OF THE INVENTION

The present invention relates to an apparatus and method for manufacturing disposable wearing articles such as paper diapers and disposable pants.

### BACKGROUND OF THE INVENTION

EP-0 405 575 discloses an apparatus for placing rubber threads around leg openings of disposable pants while crossing the rubber threads.

However, the above publication fails to disclose the relationship between the distance between the nip rolls and the guide holes (insertion distance of the rubber threads) and the placement of the rubber threads on a web.

An object of the present invention is to provide an apparatus and method for manufacturing disposable wearing articles, for example, in which an elastic member can be placed on a web along a predetermined path.

### SUMMARY OF THE INVENTION

The apparatus for manufacturing wearing articles of the present invention includes: a pair of nip rolls for nipping a first web and a first elastic member; a first moving section movable in a direction crossing the first web; and a first guide head provided on the first moving section for feeding the first elastic member at a position upstream of a position at which the first web is nipped. The radius of at least one of the pair of nip rolls is about 15 mm to about 35 mm, and a distance D between the plane including axes of the pair of nip rolls and a point at which the first guide head releases the first elastic member is about 30 mm or less.

The elastic member is swung at high speed in the direction of the axes of the nip rolls along with the movement of the guide head. Since the elastic member is pliable and low in response to the movement of the guide head due to inertial force, the elastic member is delayed after it is fed from the guide head. This delay of the elastic member fed from the guide head can be reduced by shortening the distance D. In this way, the first elastic member can be placed on the first web coated with an adhesive roughly in accordance with the pattern information.

The distance D is set longer than the distance between the plane described above and the nip position at which the first web is nipped, for avoiding the guide head from being nipped by the nip rolls. The distance may be 5 mm or more for easy maintenance of the guide head.

As the radius of the nip rolls is smaller, the guide head can be positioned deeper in the space between the nip rolls, and thus the distance D can be shorter. However, if the radius of the nip rolls is extremely small, the strength of the nip rolls against bending (rigidity) becomes small. In consideration of the above, the radius of the nip rolls is set at about 15 mm to about 35 mm. By this setting, the distance D can be small while a sufficient strength against bending is maintained.

The apparatus of the present invention may have first and second guide heads, and the first and second guide heads may be movable in the direction crossing the web without the first guide head interfering with the second guide head. In this case, the apparatus of the present invention permits two sets of elastic members to be placed crossing each other.

To shorten the distance D to about 30 mm or less, the first guide head and the second guide head may be positioned so that at least part of the first guide head and at least part of the second guide head overlap each other when the first guide head and the second guide head are projected on the plane vertical to the axes of the pair of nip rolls. The distance D can be shortened by positioning the first guide head and the second guide head so that the traces of the movements of the first and second guide heads are brought into proximity to each other.

As the elastic members, elastic threads such as rubber threads are used. Alternatively, band-like elastic members (e.g., flat rubber) may be used.

The "wearing articles" used herein include mainly disposable pants, disposable paper diapers and sanitary napkins as well as their blanks.

When the elastic members are elastic threads (e.g., rubber threads), the guide heads generally include through holes. Alternatively, they may be configured to take a U shape, a crochet needle shape or a fishhook shape.

The first elastic member may be sandwiched between the first and second webs. This fastens the first elastic member firmly.

The elastic members can be placed on the web according to a predetermined trace pattern with high precision by controlling the moving sections based on pattern information of the elastic members, movement information of the moving sections, and positional information of the webs.

According to the present invention, the "trace pattern" may be represented by the coordinates of the positions of the elastic members placed on the web.

The "positional information of the webs" may be obtained by detecting the webs, or may be information calculated from rotation information of the nip rolls.

The second guide head may be provided in addition to the first guide head, and the first and second guide heads may be positioned so that at least part of the first guide head and at least part of the second guide head overlap each other when they are projected in the direction of the axes of the nip rolls.

Alternatively, the apparatus of the present invention includes: a pair of nip rolls for nipping two webs and an elastic member between the two webs; a first moving member movable in parallel with the axes of the nip rolls as the rotation centers; a first guide head provided on the first moving member for feeding at least one elastic member between the two webs at a position upstream of a position at which the webs are nipped, a second moving member movable in parallel with the axes of the nip rolls as the rotation centers; and a second guide head provided on the second moving member for feeding at least one different elastic member between the two webs at a position upstream of the position at which the webs are nipped, wherein the first guide head and the second guide head are positioned roughly in line with each other in parallel with the axes of the nip rolls.

In the apparatus described above, the two guide heads are positioned roughly in line with each other in parallel with the axes of the nip rolls. Therefore, the distance D is the same for the two guide heads, preventing the possibility of one guide head having a distance D substantially longer than that of the other.

Note that being "positioned roughly in line with each other" means that the two guide heads are located at positions falling within a predetermined range from a straight line.

### BRIEF DESCRIPTION OF DRAWINGS

FIG. **1** is a bottom view of an apparatus for manufacturing disposable articles of Embodiment 1 of the present invention.
FIG. **2** is a front view of the apparatus of FIG. **1**.
FIG. **3** is a schematic side view of a layout of the apparatus of FIG. **1**.
FIG. **4** is a schematic perspective view of a moving mechanism of arms.
FIGS. **5A** to **5C** are bottom views showing a series of operations of guiding/feeding elastic members.
FIG. **6A** is a front view showing a trace pattern of the elastic members, FIG. **6B** is a conceptual view of an overshoot state, FIG. **6C** is a table showing the relationship between the distance bp and the height Δ, and FIG. **6D** is a graph showing the relationship in FIG. **6C.**
FIG. **7** is a perspective view of a manufacturing apparatus of Embodiment 2 of the present invention.
FIGS. **8A** and **8B** are a plan view and side view of the apparatus of FIG. **7**.
FIG. **9** is a side view of a nip roll showing the relationship between the insertion distance D and the clearance S.
FIG. **10** is an illustration of processes involved in manufacture of pants-type wearing articles from a complex web **W**_{**x**}.
FIG. **11** is an illustration of an intermediate process in manufacture of wearing articles by a longitudinal supply method.

### DESCRIPTION OF PREFERRED EMBODIMENTS

### Embodiment 1

Hereinafter, Embodiment 1 of the present invention will be described with reference to the relevant drawings.

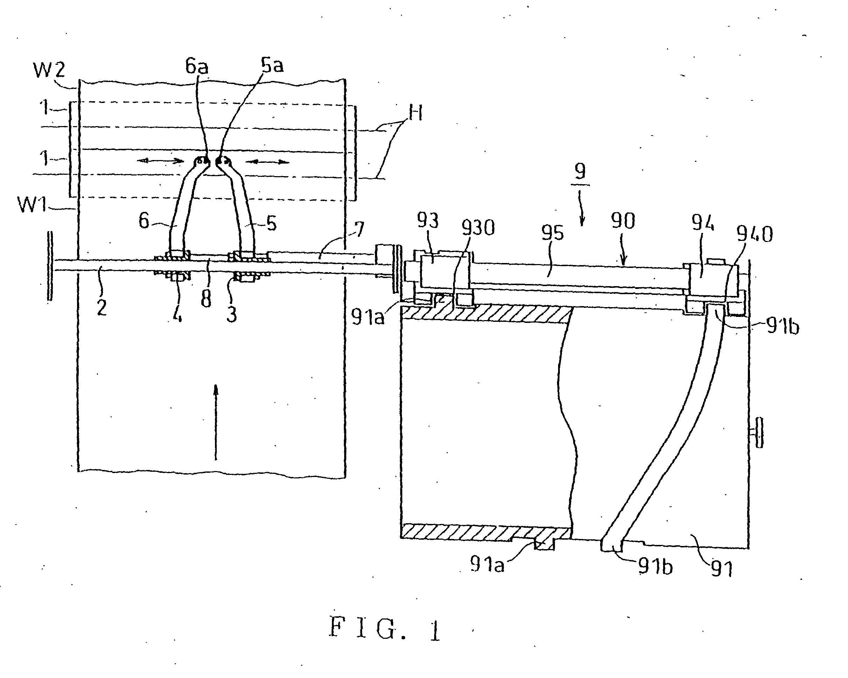

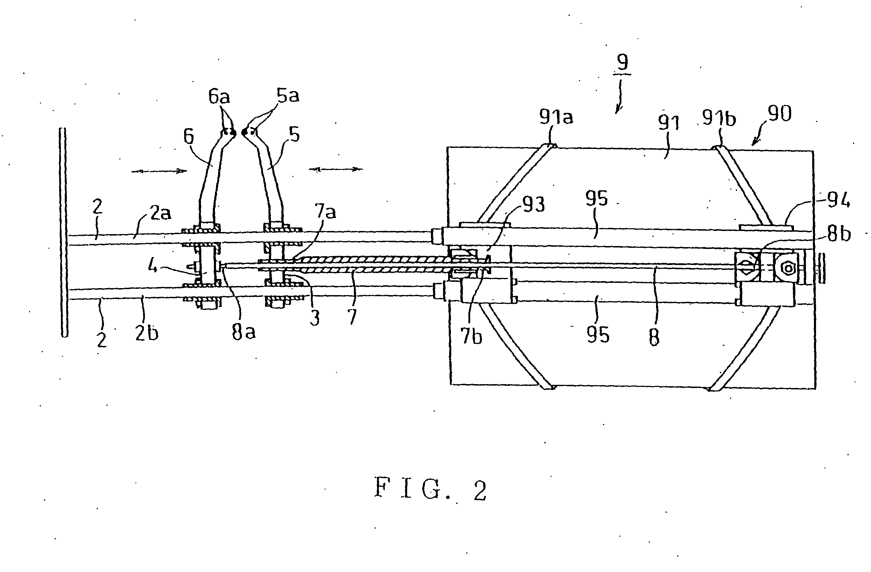

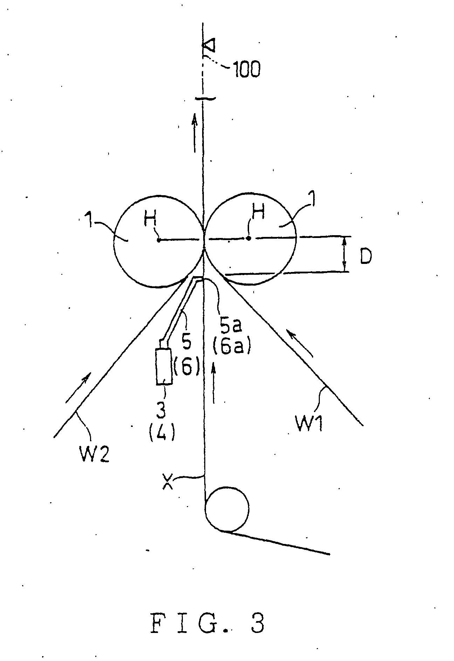

FIG. **1** is a partially cutaway bottom view of a manufacturing apparatus of Embodiment 1, FIG. **2** is a front view of the apparatus, and FIG. **3** is a schematic layout of the apparatus.

The illustrated manufacturing apparatus is an apparatus for placing two sets of elastic members X (see FIGS. **5A** to **5C**) on a first web **W1,** which is being transported in the longitudinal direction indicated by the single-ended straight arrow in FIGS. **1** and **5A** to **5C**,respectively, so that predetermined lines (paths) are traced. As shown in FIG. **3,** first and second arms (moving members) **5** and **6** are positioned near a pair of nip rolls **1, 1** which nip the first web **W1** and a second web **W2** to be bonded with the first web **W1.** Referring to FIG. **2,** the apparatus includes a guide shaft **2,** first and second sliders **3** and **4** movably attached to the guide shaft **2,** first and second arms **5** and **6** attached to the first and second sliders **3** and **4,** respectively, and a slider moving component **9** for sliding the first and second sliders **3** and **4** along the guide shaft **2** in the direction indicated by the double-ended arrows. The web used herein includes staple nonwoven, filament nonwoven, wetlaid nonwoven, drylaid nonwoven, airlaid nonwoven, airlaid pulp nonwoven, carded nonwoven, parallel-laid nonwoven, cross-laid nonwoven, random-laid nonwoven, spunlaid nonwoven, meltblown nonwoven and the like.

Hereinafter, the respective components of the apparatus will be described one by one.

The guide shaft **2** movably supports the first and second sliders **3** and **4.** As shown in FIG. **1,** the guide shaft **2** is disposed above a transport path of the first web **W1** to cross the transport path, and is composed of two generally parallel rails **2a** and **2b** as shown in FIG. **2,** for example. The guide shaft **2** is not limited to this configuration, but may be composed of a single rail having a guide groove, for example.

The first and second sliders **3** and **4** move along the guide shaft **2** to swing the elastic members **X** so that the elastic members **X** trace predetermined lines. In this embodiment, the first and second sliders **3** and **4** are spanned over the two rails **2a** and **2b** of the guide shaft **2.** An adjusting component may be provided for the first and second arms **5** and **6** and/or the first and second sliders **3** and **4** for changing the distance between the nip rolls **1, 1** and the ends of the first and second arms **5** and **6.**

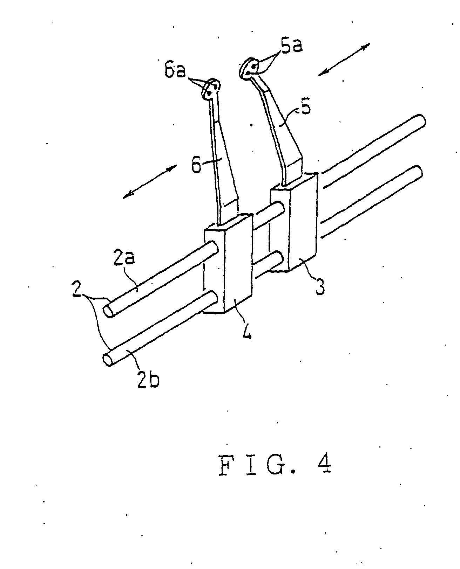

As shown in FIG. **4**, first and second guide heads (e.g., feeder heads) **5a** and **6a** are provided on the ends of the first and second arms **5** and **6,** respectively. Each of the guide heads **5a** and **6a** has one or plural through holes through which the elastic members **X** are threaded. At least one elastic member **X** is allowed to pass through each through hole. The through holes for the elastic members **X** are formed to ensure that elastic threads constituting the elastic members **X** are basically guided and fed separately to the webs **W1** and **W2** without tangling with each other when the elastic members **X** are curved forming wavelike lines in association with the movement of the arms **5** and **6** (see FIG. **4**). The first and second arms **5** and **6** together with the respective first and second guide heads **5a** and **6a** may form a T shape, resembling the shape of a safety razor, or form an L shape. The inner diameter of the through hole may be 0.5 mm to 5mm. In the case that the through hole is elongate, the inner diameter refers to the major diameter.

The first and second guide heads **5a** and **6a** shown in FIG. **1** are positioned apart from each other in the direction of axes **H** of the nip rolls **1, 1** as the rotation center, and movable roughly in parallel with the axes **H** in association with the movement of the sliders **3** and **4,** respectively, to which the arms **5** and **6** are attached, as will be described later. By the movement of the arms **5** and **6,** the separate elastic members **X** are fed between the webs **W1** and **W2** upstream of the position at which the webs **W1** and **W2** are nipped (nip position). The two arms are not necessarily required, but a single arm may be provided.

As shown in FIG. **3,** during the feeding of the elastic member **X** to the web **W1,** an insertion distance D, which is the distance between the plane including the axes **H** of the pair of nip rolls **1, 1** and the position of the guide head **5a, 6a** (the point at which the elastic member **X** is released), is preferably set at 30 mm or less in consideration of speedup. With the insertion distance D of 30 mm or less, influence of the delay of the elastic member **X** can be reduced, and thus the elastic member **X** can be placed on the first web **W1** at higher speed while being curved correctly.

The slider moving component **9** shown in FIG. **2** enables the first and second sliders **3** and **4** to slide along the guide shaft **2** so that the elastic members **X** can trace wavelike lines. In this embodiment, the slider moving component **9** includes a first drive shaft **7,** a second drive shaft **8** and a cam mechanism **90.** The cam mechanism **90** includes a cylindrical cam **91,** a first slider block **93,** a second slider block **94** and a guide rail **95**.

The first drive shaft **7,** which is a cylinder, transmits a driving force of the slider moving component **9** to the first slider **3.** An end **7a** of the first drive shaft **7** is secured to the first slider **3** and the other end **7b** is secured to the first slider block **93.** The second drive shaft **8,** which is a rod, penetrates through the first drive shaft **7** in the present example movably backward and forward in the direction of the axis of the first drive shaft **7,** and transmits a driving force of the slider moving component **9** to the second slider **4.** An end **8a** of the second drive shaft **8** is secured to the second slider **4** and the other end **8b** is secured to the second slider block **94.** The first and second drive shafts **7** and **8** run in the space between the rails **2a** and **2b** constituting the guide shaft **2**.

The cylindrical cam **91** forces the first and second slider blocks **93** and **94** to reciprocate. On the circumference of the cylindrical cam **91** of the present example, there are formed a pair of convex guides **91a** and **91b** curved according to the desired movement patterns of the first and second sliders **3** and **4,** respectively. Alternatively, grooves may be formed according to the movement patterns. The first and second slider blocks **93** and **94** may be moved with a servomotor, for example.

The first and second slider blocks **93** and **94** transmit the driving force of a motor to the first and second drive shafts **7** and **8,** respectively, as they move on the guide rail **95** following the guides **91a** and **91b** of the cylindrical cam **91.** The first and second slider blocks **93** and **94** have cam followers **930** and **940** engaging with the guides **91a** and **91b** on the circumference of the cylindrical cam **91** as shown in FIG. **1.**

The guide rail **95,** which movably supports the first and second slider blocks **93** and **94** as described above, is disposed as an extension from the guide shaft **2** in the present example.

Hereinafter, an example of the operation of the apparatus in Embodiment 1 will be described.

FIGS. **5A** to **5C** are views illustrating exemplary traces of the elastic members **X, X** to be sandwiched between the webs **W1** and **W2**. In these figures, only the first web **W1** is shown, omitting the second web **W2** to be bonded with the first web **W1** and the nip rolls **1, 1** for nipping these webs for convenience.

Referring to FIG. **5A,** the first and second sliders **3** and **4** are farthest from each other on the guide shaft **2** when the first and second slider blocks **93** and **94** are located closest to each other. The first and second slider blocks **93** and **94** are farthest from each other at the position where the spacing between the guides **91a** and **91b** of the cylindrical cam **91** is narrowest.

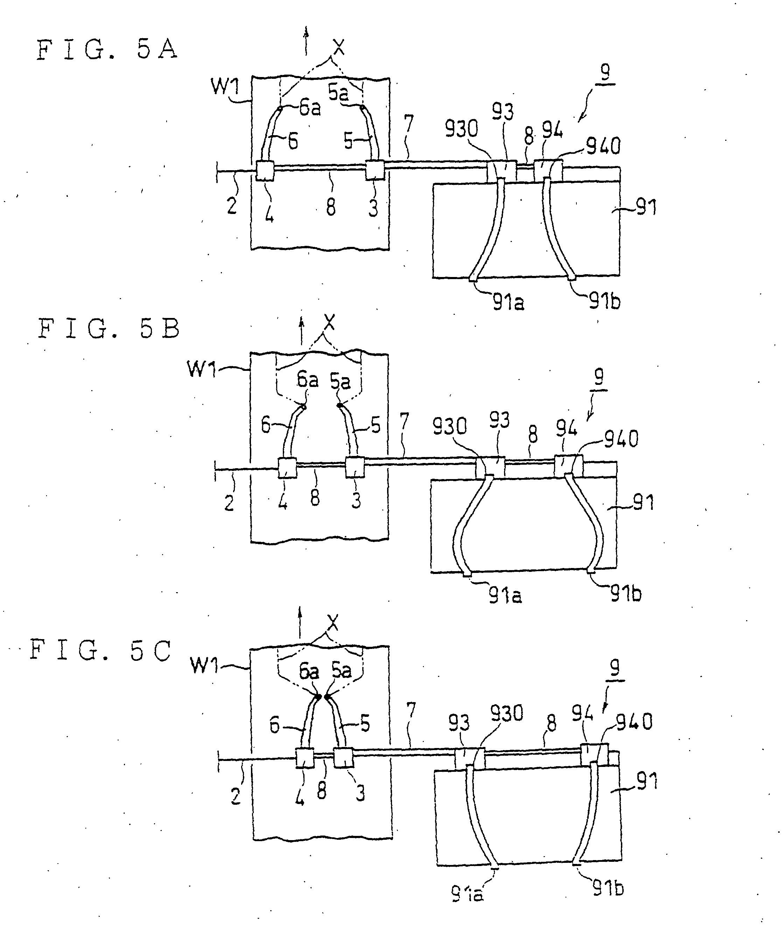

In the above state, when a motor (not shown) is activated to rotate the cylindrical cam **91,** the spacing between the guides **91a** and **91b** of the cylindrical cam **91** is gradually widened as shown in FIG. **5B.** Following this, the first and second slider blocks **93** and **94** gradually move apart from each other along the guide rail **95** (FIG. **1**). The movement of the first and second slider blocks **93** and **94** is transmitted to the first and second sliders **3** and **4** via the first and second drive shafts **7** and **8,** respectively, to allow the first and second sliders **3** and **4** to gradually come closer to each other. Along with this movement, the elastic members **X, X** gradually come closer to each other toward the center of the web **W1.**

As the cylindrical cam **91** is further rotated, as shown in FIG. **5C,** the spacing between the guides **91a** and **91b** of the cylindrical cam **91** finally reaches the maximum. At this point, the first and second sliders **3** and **4** are closest to each other.

As the cylindrical cam **91** is continuously rotated, the first and second slider blocks **93** and **94** and the first and second sliders **3** and **4** follow the operation described above in reverse, that is, operate in the order of FIG. **5B** and FIG. **5A.** With this operation, the elastic members **X, X** are gradually apart from each other toward the edges of the web **W1.** Note that the movement of the first and second arms **5** and **6** shown in FIGS. **5A** to **5C** is merely an example, and it is determined depending on the shape of the guide heads.

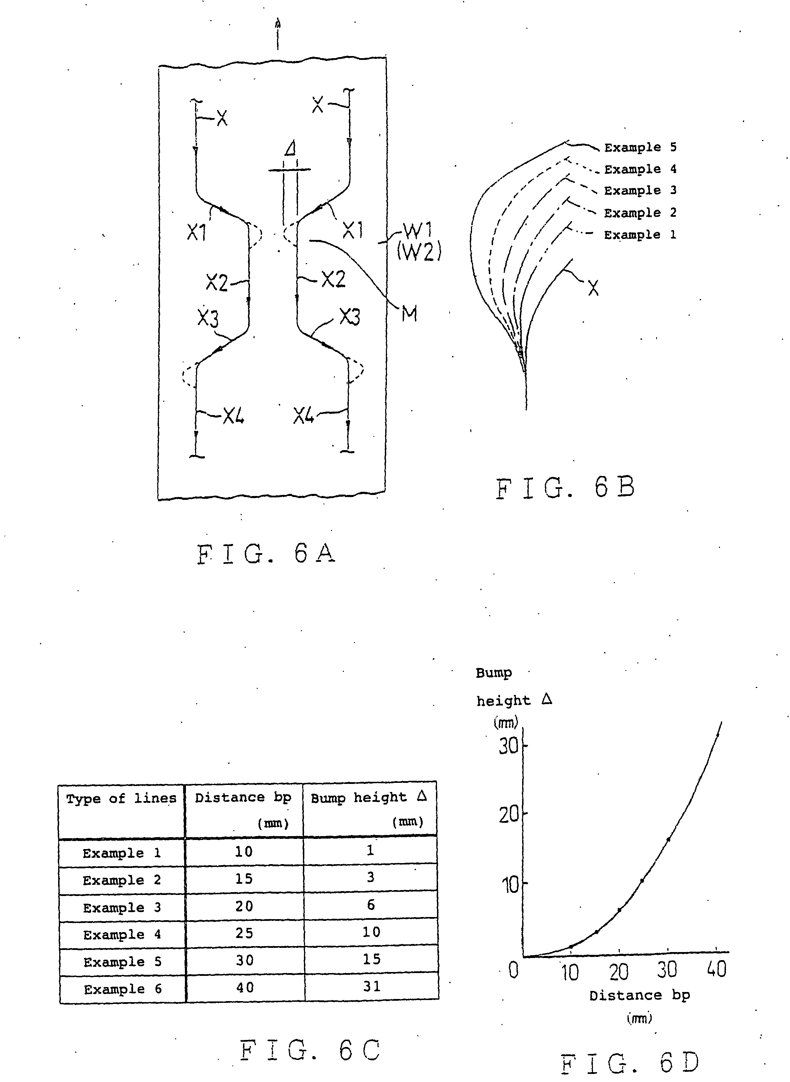

Thereafter, the operation described above is repeated, so that the elastic members **X, X** are fed on the first web **W1** tracing predetermined wavelike lines as shown by X1, X2, X3 and X4 in FIG. **6A,** for example.

The solid lines in FIG. **6A** represent the elastic members placed on the web. The elastic members will fail to be placed as shown by the solid lines X1 to X4 if the guide heads **5a** and **6a** travel as represented by the solid lines X1 to X4. The reason is that the elastic members are delayed behind the movement of the guide heads **5a** and **6a.**

To cancel the delay of the elastic members, the guide heads **5a** and **6a** in the present example are configured to overshoot the desired elastic member location. In FIG. **6A,** positions at which the guide heads **5a** and **6a** are especially greatly overshot are diagrammatically shown by the dashed lines. Note that FIG. **6A** shows the movement of only one through hole for the elastic member for each of the guide heads **5a** and **6a,** omitting the remaining through holes, for simplification of description.

FIG. **6B** is an enlarged view of one portion of the dashed line in FIG. **6A.** In FIG. **6B,** the bold solid line X represents the trace of the elastic member placed on the web, and the other lines represent traces of the guide head **5a, 6a** required to place the elastic member on the bold solid line, obtained by simulation. Since the web is being transported with respect to time, the traces of the guide heads **5a** and **6a** also move in the direction of the transport of the web relative to the web. For reference, FIGS. **6C** and **6D** show the results of simulation on the relationship between the height Δ from the line part X2 in the steady state as the maximum overshoot (hereinafter, referred to as the "bump height Δ") and the distance bp between the guide heads **5a** and **6a** and the nip position.

The distance bp and the insertion distance D may be generally considered equal to each other, but to be strict, the insertion distance D is normally longer than the distance bp by about 1 mm to about 5 mm due to factors such as the thicknesses of the webs **W1** and **W2**, the elastic members and the like even if the points at which the elastic members **X** are released are the same.

The delay of the elastic members **X** from the guide heads **5a** and **6a** is smaller as the distance bp between the guide heads **5a** and **6a** and the nip position is shorter. When the distance bp = 40 mm, the bump height Δ = 31 mm, which is about twice as large as that observed when the distance bp = 30 mm. It is found therefore that the bump height Δ can be effectively reduced by setting the distance bp at 30 mm or less (insertion distance D ≦ 35 mm). In other words, by this setting, the distance of movement of the guide heads **5a** and **6a** needed to produce the desired pattern (e.g., as illustrated in FIG. **6A**) is shortened, and the resultant manufacturing apparatus is suitable for high-speed operation. When the distance bp ≦ 25 mm (insertion distance D ≦ 30 mm), the bump height Δ can be considerably small. This is very advantageous because the load of the apparatus can be further reduced. Thus, by shortening the distance bp, the simulation of the traces of the guide heads **5a** and **6a** is no longer necessary for placement of the elastic members in some cases.

If the distance bp is greater than 40 mm, the resultant apparatus is not suitable for mass production of disposable articles. One reason is that the load applied to the apparatus is great. As another reason, the distance which the guide heads must travel becomes too long.

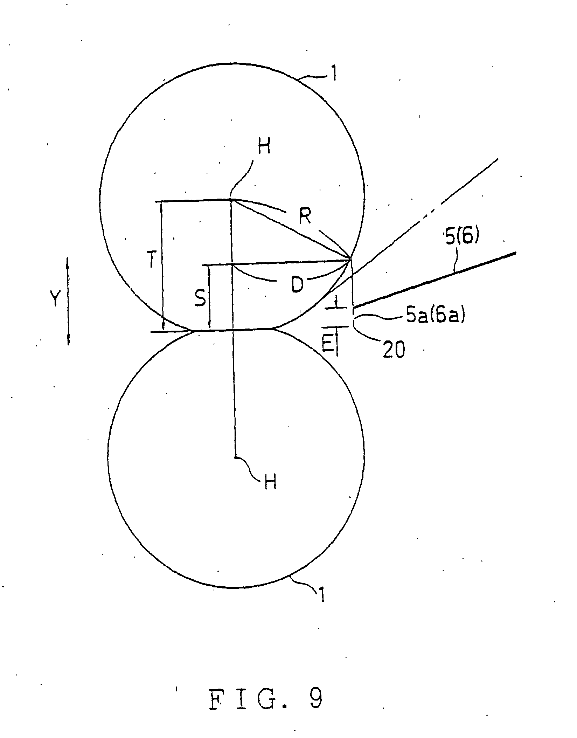

Referring to FIG. **9,** the relationship between the insertion distance D and the width E of an end **20** of the guide head **5a, 6a** in the Y direction will be described. The Y direction herein refers to the direction of the straight line connecting the axes H of the nip rolls **1, 1.** When the radius of the nip rolls **1, 1.** is R, the distance between the axes H of the nip rolls **1, 1** is 2T, and the maximum of the clearance between the nip rolls **1, 1** allowing the end **20** to enter is 2S, wherein the relationship S = T - (R² - D²)^{1/2} is derived. Note that FIG. **9** shows the state where the nip rolls **1, 1** are pressed against each other, that is, the state T < R. However, in consideration of the thicknesses of the webs and the elastic member, the relationship in the state T ≧ R may also be presumed.

When the guide heads **5a** and **6a** are not positioned to overlap each other in the direction of the axes H but can travel crossing each other, the width E of the end **20** should be less than T - (R² - D²)^{1/2} when R - D ≧ 0. When the guide heads **5a** and **6a** are positioned to completely overlap each other in the direction of the axes H, the width E of the end **20** should be less than 2T - 2(R² - D²)^{1/2} when R - D ≧ 0. When R - D < 0, the ends **20** of the guide heads **5a** and **6a** are outside the clearance between the nip rolls **1, 1,** and thus no limitation is basically required for the width E of the ends **20** of the guide heads **5a** and **6a.**

The insertion distance D is desirably as short as possible as described above. However, when R > T, the minimum insertion distance D is greater than (R² - T²)^{1/2}.

As the curvature of the nip rolls **1, 1** is greater (as the radius thereof is smaller), the clearance between the nip rolls **1, 1,** that is, the space allowing the ends **20** to enter is greater. However, as the radius of the nip rolls **1, 1** is smaller, the strength against bending is weaker. With weak strength, the nip rolls may be deformed causing instability in the peripheral speed of the nip rolls and thus variation in the transport speed of the webs. As a result, creases may be disadvantageously generated in the webs. To avoid this problem, the radius R of the nip rolls **1, 1** is preferably 15 mm or more when the nip rolls **1, 1** are made of carbon steel for machine structure, rolled steel for general structure and the like. The radius of the nip rolls can be further small if the nip rolls are made of a type of steel with which distortion is smaller during the nipping of the elastic members between the webs than the above steel types. Alternatively, the strength of the nip rolls can be increased by using nip rolls produced by quenching a plurality of cylindrical members having different sizes and placing one cylindrical member around another without a gap therebetween to form one roll.

On the contrary, as the curvature of the nip rolls **1, 1** is smaller (as the radius thereof is greater), the clearance between the nip rolls **1, 1** is smaller compared with the case of a greater curvature. This blocks the attempt of shortening the insertion distance D. Moreover, as the radius of the nip rolls **1, 1** is greater, the mass of the nip rolls **1, 1** increases. This generates the necessity of increasing the frame strength of the manufacturing apparatus, and thus fails to provide a compact manufacturing apparatus. For the reasons described above, the radius of the nip rolls **1, 1** is preferably 40 mm or less.

Suppose the guide heads **5a** and **6a** are positioned so that they can travel crossing each other, the radius of the nip rolls **1, 1** is 40 mm or less, the insertion distance D is 30 mm, and the allowance of the distance of the ends **20** of the guide heads **5a** and **6a** from the nip rolls **1, 1** (webs) is about 5 mm. Then, the clearance S is about 11 mm. Therefore, it is ensured that the guide heads **5a** and **6a** each having a through hole with a radius of 5 mm can be used. The above allowance refers to a value to be set to prevent the guide heads **5a** and **6a** from being in contact with the webs or being deformed, for example. In particular, it is disadvantageous for the guide heads **5a** and **6a** to be in contact with an adhesive applied to the webs. The allowance should empirically be in the range of about 1 mm to about 15 mm, but may vary a little depending on the thickness of the adhesive applied to the webs, dropping of the adhesive, slack of the webs, and the angle at which the webs are introduced to the nip rolls **1, 1.**

When the guide heads **5a** and **6a** are positioned to roughly overlap each other in the direction of the axes H, the insertion distance D can be further reduced compared with the case of positioning the guide heads **5a** and **6a** so that they can travel crossing each other.

The radii of the pair of nip rolls **1, 1** may be the same or different from each other. In the case of the guide heads **5a** and **6a** roughly overlapping each other in the direction of the axes H, the insertion distance D can be made 30 mm or less even when the radius of one nip roll is more than 35 mm as long as the radius of the other nip roll is equal to or less than 35 mm.

In the example described above, the guide heads **5a** and **6a** were assumed to be apart from the webs in the Y direction by about 5 mm. However, if no binder is applied on one web, the insertion distance D can be further reduced by shifting the position of the guide heads **5a** and **6a** toward the web to which no binder is applied.

Alternatively, the distance of the guide heads **5a** and **6a** from the webs can be controlled and thus reduced by applying the adhesive thinly and uniformly and/or applying tension to the webs to prevent the webs from slacking. As a result, the insertion distance D can be further reduced.

### Embodiment 2

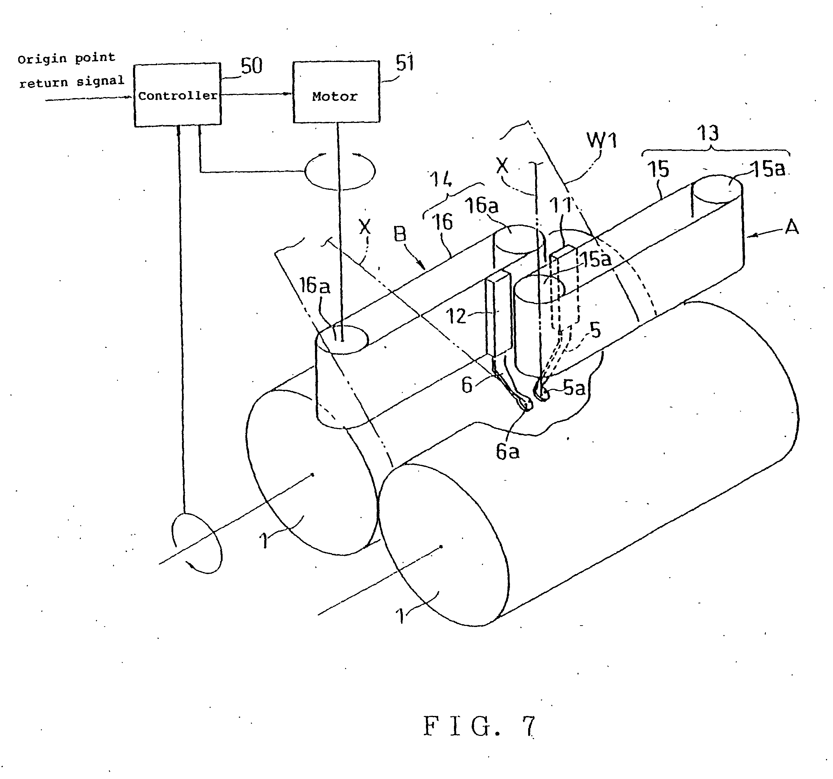

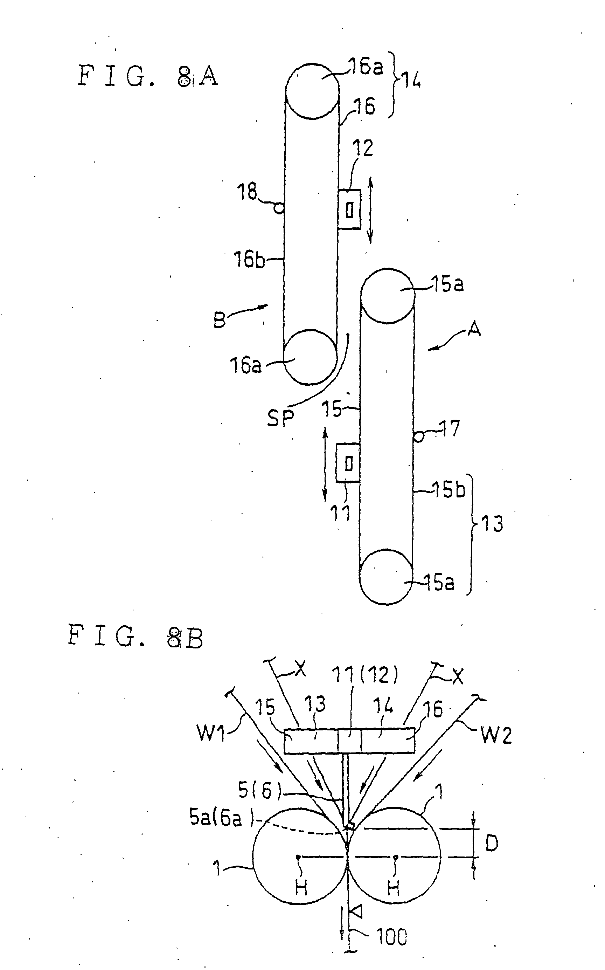

FIGS. **7, 8A** and **8B** illustrate a manufacturing apparatus of Embodiment 2 of the present invention.

Referring to FIG. **7**, the apparatus of this embodiment includes a first mount component **A** and a second mount component **B.** The first mount component **A** includes a first mover **13** and a first arm **5** for guiding at least one elastic member **X** to a position near the contact position of nip rolls **1, 1.** The second mount component **B** includes a second mover **14** and a second arm **6** for guiding at least one elastic member X to a position near the contact position of nip rolls **1, 1.** The first mover **13** forces the first arm **5** to reciprocate in the direction of the width of a first web **W1.** The second mover **14** forces the second arm **6** to reciprocate in the direction of the width of a second web **W2.**

Referring to FIG. **8A,** each of the movers **13** and **14** includes a pair of pulleys **15a, 15a (16a, 16a)** and a belt **15 (16)** looped over the pair of pulleys **15a, 15a (16a, 16a).** One of the pair of pulleys **16a, 16a** of the second mover **14** is rotated with a motor **51** shown in FIG. **7.** Although not shown, the first mover **13** is also rotated with a similar motor. The motor **51** is controlled by a controller **50** placed inside or outside the manufacturing apparatus. First and second fittings **11** and **12** are mounted on the belts **15** and **16,** respectively, so that they are reciprocated in the direction of the width of the webs **W1** and **W2**.

The belts **15** and **16** are preferably light in weight. The lightweight belts **15** and **16** can reduce the influence of inertial force. For example, when the apparatus manufactures general disposable pants or diapers, the mass of one belt **15** or **16** together with the arm **5** or **6** is preferably 1 kilogram or less. Considering the strength of the belt **15** or **16** and the arm **5** or **6,** the mass is preferably 50 to 200 grams.

By adopting a servomotor as the motor **51,** the rotational speed of the motor and the like can be easily controlled. Alternatively, an induction motor may be adopted for easy maintenance of the motor **51.**

Each motor **51** may be directly coupled to the pulley **15a** or **16a.** Alternatively, it may be disposed separately at a position near the ends of the nip rolls **1, 1,** or at a position closer to the center of the nip rolls **1, 1** in the width direction. It is also possible to drive both pulleys with a motor.

The controller **50** controls the rotational speed, rotational acceleration and the like of the motor **51.** This control by the controller **50** may be feedforward control, feedback control, fuzzy control, optimal control, neuro control, robust control and the like. For example, in feedback control, the controller **50** performs sampling of trace information (pattern information) of the elastic members **X** as shown in FIG. **6A** and rotation information (movement information) of the motor **51** in FIG. **7** including at least one of the rotational speed and rotational acceleration of the motor **51,** for example. Based on the sampled data, the controller **50** controls the voltage applied to the motor **51,** the current flow to the motor **51** and/or the frequency thereof.

When the elastic members are placed in a cyclic fashion (for example, in curved lines as shown in FIG. **6A**), positional information of the webs **W1** and **W2** may be fed back to the controller **50** to prevent displacement of the elastic members **X** relative to the webs **W1** and **W2** in the direction of transport of the webs **W1** and **W2.** The positional information of the webs **W1** and **W2** may be obtained by actually measuring the transport of the webs **W1** and **W2** with a sensor (an infrared sensor, an ultrasonic sensor or an air sensor), may be calculated from the rotation information of the nip roll **1,** or may be determined based on both the measured value from the sensor and the rotation information to enhance the precision. The rotation information of the nip roll **1** may be measured directly or indirectly via an encoder or the like and sent to the controller **50.** The controller **50** generates a control signal for the motor **51** based on the positional information of the webs **W1** and **W2,** the trace information of the elastic members **X** and the rotation information of the motor **51,** and sends the control signal to the motor **51.** The motor **51** rotates based on the control signal, and rotational energy of the motor **51** is transmitted to a second guide head **6a** directly or indirectly. A first guide head **5a** is also controlled in the same manner as the second guide head **6a.**

The controller **50** may also receive an origin point return signal. The origin point is a predetermined point on the route of the second guide head **6a** traveling across the web, at which a new period starts. That is, the controller **50** forces the second guide head **6a** to return to the origin point upon receipt of the origin point return signal when the second guide head **6a** is located at a position other than the origin point. This permits synchronization of this process with another machining process performed for the webs **W1** and **W2** upstream or downstream of the manufacturing apparatus. When this apparatus is incorporated in a system for manufacturing disposable wearing articles, the machining process performed for the webs **W1** and **W2** upstream or downstream includes a process of placing absorbents on the web at a predetermined pitch, a process of making openings for wearer's legs through the web at a predetermined pitch, and the like.

Although the control of the second guide head **6a** was described above, the first guide head **5a** may also be controlled in the same manner as the second guide head **6a.**

The manufacturing apparatus may be provided with a vibration absorber. For example, when the apparatus is driven at high speed, the side of the belt **15, 16** opposite to the side on which the arm **5, 6** is mounted (non-mount side) may vibrate. In such an occasion, the vibration absorber can attenuate vibration at the belt **15, 16.**

As an example of the vibration absorber, at least one balancer (not shown) may be provided on the non-mount side **15b, 16b** of the belt **15, 16.** The balancer may be provided on the inner circumference of the belt **15, 16,** but the degree of freedom at design of the manufacturing apparatus is greater when the balancer is provided on the outer circumference of the belt. Such balancers may be provided both on the inner and outer circumferences of the belt. The entire mass of the balancer(s) may be the same as that of the arm **5, 6.**

As another example of the vibration absorber, at least one contact **17, 18** may be placed in contact with the belt **15, 16** on the non-mount side **15b, 16b.** For example, at least one contact **17, 18** may be placed in contact with the outer circumference of the belt **15, 16,** or the inner circumference of the belt **15, 16.**

As the contact **17, 18,** a simple plate or a highly elastic member such as a sponge may be used, but more preferably a roller may be used.

Alternatively, the vibration absorber may be mounted on the side of the belt **15, 16** on which the arm **5, 6** is mounted.

Each of the first and second arms **5** and **6** has at least one guide head **5a, 6a** for guiding the elastic member **X** to a position near the contact position at which the nip rolls **1, 1** come into contact with each other. As in the construction of the manufacturing apparatus of Embodiment 1 shown in FIG. **3**, the centers of the guide heads **5a** and **6a** may be positioned roughly in line with each other as shown in FIG. **7**. The guide heads **5a** and **6a** may be positioned generally in one virtual plane **100** (plane in which the pair of nip rolls **1, 1** are in contact with each other) shown in FIG. **8B.** The above wording "generally" implies that there is included the case that the guide heads **5a** and **6a** are not completely in the plane **100.**

FIG. **8B** is a side view of the apparatus of Embodiment **2.** Two sets of elastic members **X, X** are introduced to the contact portion of the two nip rolls **1, 1.** In this embodiment, the first and second guide heads **5a** and **6a** are positioned to overlap each other when viewed in the direction of the axes H of the nip rolls **1, 1,** not displacing from each other in the direction of the line connecting the axes H of the nip rolls **1, 1** (lateral direction in FIG. **8B**). Therefore, the first and second guide heads **5a** and **6a** can be positioned nearer to the contact position of the two nip rolls **1, 1,** compared with the case of displacing the guide heads **5a** and **6a** from each other.

Alternatively, the pair of movers **13** and **14** may be placed facing each other, and the first and second fittings **11** and **12** may travel in a space SP between the movers **13** and **14** as illustrated in FIG. **8A.**

The belts **15** and **16** may be displaced from each other in the tangential direction of the nip rolls **1, 1** (vertical direction in FIG. **8B**). In this case, the fittings **11** and **12** are mounted on the belts **15** and **16** at positions vertically displaced from each other. Therefore, the fittings **11** and **12** can be displaced largely in the width direction (lateral direction), and thus the width of the fittings **11** and **12** can be made larger than the space between the two belts **15** and **16.**

The elastic members **X** may be supplied to the guide heads **5a** and **6a** through the insides of the belts **15** and **16.**

In the manufacturing apparatus shown in FIG. **7** and FIGS. **8A** and **8B,** the motor **51** drove the belt. Alternatively, the motor **51** may drive a drive shaft such as the drive shaft **7, 8** shown in FIG. **2,** to place the elastic members. To convert the rotational movement to the reciprocating movement, a crank mechanism, a gear, a ball screw, a cam, or a combination thereof may be used.

In Embodiments 1 and 2, an adhesive was applied to at least one of the webs **W1** and **W2**. The application may be continuously or intermittently. When a hotmelt binder is used, it may be applied by beading, coating with a coater, spiral coating, curtain coating, spraying, coating with a transfer roll or the like. As the hotmelt, a synthetic rubber type or an olefin type may be used. The elastic members may be coated with an adhesive as described above.

In Embodiments 1 and 2, two sets of elastic members **X** were sandwiched between the first and second webs **W1** and **W2.** Alternatively, one elastic member set **X** may be fed from the first guide head to be sandwiched between the first and second webs **W1** and **W2**.

In Embodiments 1 and 2, the first web **W1** and at least one elastic member set **X** may be nipped between the nip rolls **1, 1.** In this case, the surface of the nip roll **1** coming into contact with the side of the web **W1** coated with the adhesive is preferably covered with a highly releasable material. As the highly releasable material, silicone and fluorocarbon resins (for example, TEFLON from DuPont) may be used. For example, the nip roll **1** may be wound with silicone rubber or coated with TEFLON. The use of one web with an elastic member adhering thereto as described above can reduce the material cost compared with the use of two webs sandwiching an elastic member.

Embodiment 1 and 2 described above may be applied to the manufacture of disposable wearing articles. When the disposable wearing articles are disposable diapers, leg gathers of the diapers may be formed by implementing any of the embodiments of the present invention. When fit gathers for the diapers are curved, they may be formed by implementing any of the embodiments of the present invention.

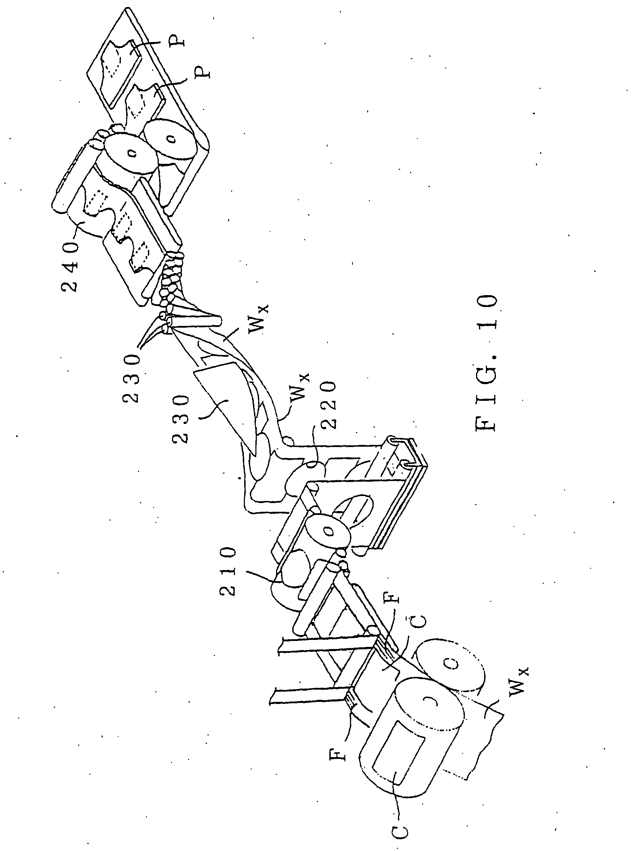

Hereinafter, an example of manufacture of disposable wearing articles by implementing Embodiment 2 will be described with reference to FIGS. **7** and **10.**

As described above, at least one of the two webs **W1** and **W2** is coated with an adhesive continuously or intermittently. The coated webs **W1** and **W2** are transported to the nip rolls **1, 1.** The guide heads **5a** and **6a** having through holes with a diameter of about 0.5 mm to about 5 mm are moved in the direction crossing one of the webs (**W1**) with the motor **51** located upstream of the nip rolls **1, 1** shown in FIG. **7.** The controller **50** controls the motor **51** based on pattern information of the elastic members **X** to be placed on the web **W1,** driving information of the motor **51,** and positional information of at least one of the webs **W1** and **W2**. The elastic members **X** are guided to positions between the two webs **W1** and **W2,** and then integrated therewith by the pair of nip rolls having a radius of about 15 mm to about 35 mm. Thus, formed is a complex web **W**_{**x**} which includes portions to be used as leg gathers of pants-type diapers, if the wearing articles are pants-type diapers, formed by the elastic members. Subsequently, as shown in FIG. **10,** absorbents **C** are placed on the complex web **W**_{**x**}**,** and openings **220** for wearer's legs are then formed through the complex web **W**_{**x**} with a die cutter **210.** The openings for legs may be formed before the placement of the absorbents **C.**

The openings for legs may otherwise be formed before the elastic members **X** are sandwiched between the two webs **W1** and **W2**.

The absorbents **C** may include a highly absorptive polymer containing at least one of pulp, an acrylic acid ester and a water-soluble polysaccharide. The absorbents **C** may also include an airlaid member. The complex web **W**_{**x**} on which the absorbents **C** are placed is then folded in a folding section **230** so that the two edges of the complex web **W**_{**x**} are put together in alignment. In a cut/seal section **240,** the folded complex web **W**_{**x**} is sealed for partitioning the complex web **W**_{**x**} into wearing articles, and the complex web **W**_{**x**} partitioned with the sealing is cut, to complete wearing articles **P.**

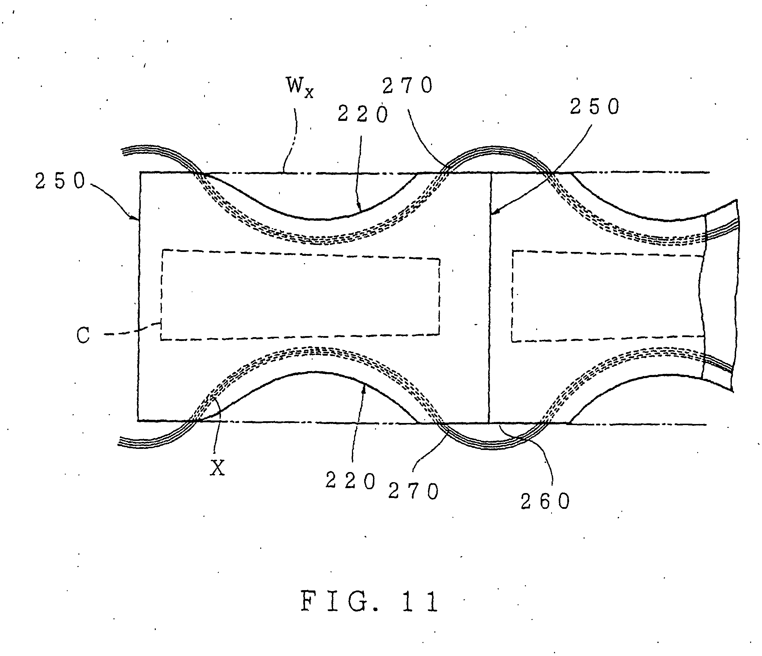

In the method for manufacturing wearing articles described above, the absorbents **C** were supplied laterally. Wearing articles can also be manufactured by supplying the absorbents **C** longitudinally. For example, as shown in FIG. **11,** openings **220** for wearer's legs are formed through the complex web **W**_{**x**} and then the absorbents **C** are placed on the complex web **W**_{**x**}**.** The step of forming the openings **220** and the step of placing the absorbents **C** may be in reverse order.

The complex web **W**_{**x**} with the absorbents **C** placed thereon is cut at a predetermined length (pitch) with a cutter. The cut pieces are folded so that cut edges **250** are put together in alignment as required. When the wearing articles are of a pants type, the sides **260** of the folded pieces of the complex web **W**_{**x**} are sealed excluding the openings **220.** To state differently, the cut pieces of the complex web **W**_{**x**} are folded so that the cut edges **250** are put together in alignment. At least two portions of each folded piece are sealed to thereby form at least three openings (for the waist and two legs of the wearer). Note that portions **270** of the elastic members **X** are actually located near the sides **260** of the complex web **W**_{**x**} due to the shrinking force of the elastic members X. The portions **270** may be cut during the cutting of the complex web **W**_{**x**}**,** and removed from the complex web **W**_{**x**} due to heat during the heat-sealing of the sides **260.**

The wearing articles may have fit gathers **F** as shown in FIG. **10.** Such fit gathers may be curved. For example, curved fit gathers as described in Japanese Laid-Open Patent Publication No. 7-299094 which is hereby incorporated by reference may be formed together with the leg gathers by the apparatus of the present invention. If curved fit gathers are to be formed for diapers having no leg gathers, a pattern for placing fit gathers may be used in place of the pattern for placing leg gathers described above.

Thus, according to the present invention, in the apparatus for manufacturing disposable wearing articles, the distance D between the plane including the axes of the pair of nip rolls and the point at which the first guide head releases the elastic member is set at 30 mm or less. With this distance D set short, even when the elastic member fed from the guide head is swung in the direction of the width of the web at high speed, the elastic member is prevented from delaying substantially behind the guide head. Thus the elastic member can be placed on the web along a predetermined path.

In addition, by using nip rolls having a small radius, the space formed between the nip rolls is widened. A guide head having a smaller diameter can be positioned deep in the widened space. In this way, since the distance D can be reduced, delay of the elastic member is prevented. Thus, the elastic member can be placed on the web along a predetermined path.

The precision of the placement of the elastic members improves by controlling the movement of the drive sections based on pattern information of the elastic members, movement information of the moving sections, and positional information of the webs.

By positioning at least part of the first guide head and at least part of the second guide head in line with each other in parallel with the axes of the nip rolls, the two guide heads can be positioned nearer to the contact position at which the nip rolls come into contact with each other.

## Claims

1. An apparatus for manufacturing wearing articles, comprising:
a pair of nip rolls for nipping a first web and a first elastic member;
a first moving section movable in a direction crossing the first web; and
a first guide head formed on the first moving section for feeding the first elastic member at a position upstream of a position at which the first web is nipped,
wherein the radius of at least one of the pair of nip rolls is 15 mm to 35 mm, and
a distance D between a plane including axes of the pair of nip rolls and a point at which the first guide head releases the first elastic member is 30 mm or less.

2. The apparatus according to claim 1, wherein the first guide head feeds the first elastic member between the first web and a second web, and
the pair of nip rolls nip the first web, the first elastic member and the second web.

3. The apparatus according to claim 1 or 2, further comprising:
a second moving section movable in a direction crossing the first web; and
a second guide head formed on the second moving section for feeding a second elastic member at a position upstream of the position at which the first web is nipped,
wherein at least part of the first guide head and at least part of the second guide head overlap each other when the first guide head and the second guide head are projected on a plane parallel to the axes of the pair of nip rolls.

4. The apparatus according to any of claims 1 to 3, further comprising a controller for controlling movement of the first moving section based on pattern information indicating a trace pattern of the elastic member placed on the first web, movement information of the first moving section, and positional information of the first web.

5. The apparatus according to claim 1 or 2, further comprising:
a second moving section movable in a direction crossing the first web;
a second guide head formed on the second moving section for feeding a second elastic member at a position upstream of the position at which the first web is nipped; and
a controller for controlling the movement of the first moving section and the second moving section based on at least one of pattern information indicating a trace pattern of the elastic members placed on the first web, movement information of the first moving section, movement information of the second moving section, positional information of the first web, and positional information of a second web.

6. A method for manufacturing wearing articles, comprising the steps of:
applying a binder to at least one of two webs continuously or intermittently;
reciprocating a guide head having at least one through hole with a power section in a direction crossing at least one of the two webs while feeding an elastic member between the two webs via the through hole;
controlling the power section based on at least one of pattern information indicating a pattern of the elastic member placed on the web, movement information of the power section, and positional information of at least one of the two webs;
nipping the two webs and the elastic member therebetween between a pair of nip rolls to form a complex web, at least one of the pair of nip rolls having a radius of 15 mm to 35 mm;
forming leg openings through the two webs or the complex web;
placing absorbents on the complex web; and
cutting the complex web with the absorbents placed thereon,
wherein in the step of reciprocating the guide head, the guide head is reciprocated keeping a distance between a point at which the guide head releases the elastic member and a plane including axes of the nip rolls at 30 mm or less.

7. The method according to claim 6, further comprising the steps of:
folding the complex web with the openings formed therethrough and the absorbents placed thereon so that the edges of the complex web are put together in alignment; and
partitioning the folded complex web with sealing before cutting the complex web.

8. The method according to claim 6, further comprising the steps of:
folding cut pieces of the complex web so that cut edges of the complex web are put together in alignment; and
sealing at least two portions of each of the folded cut pieces of the complex web so that the folded cut piece has at least three openings.

## Patentansprüche

1. Vorrichtung für die Herstellung von Bekleidungsartikeln, die umfasst:
ein Paar Presswalzen, um eine erste Bahn und ein erstes elastisches Element zusammenzupressen;
einen ersten Bewegungsabschnitt, der in einer Richtung quer zu der ersten Bahn beweglich ist; und
einen ersten Führungskopf, der an dem ersten Bewegungsabschnitt ausgebildet ist, um das erste elastische Element an eine Position stromaufseitig von einer Position, an der die erste Bahn gepresst wird, zuzuführen,
wobei der Radius wenigstens einer der beiden Presswalzen im Bereich von 15 mm bis 35 mm liegt und
ein Abstand D zwischen einer Ebene, die die Achsen der beiden Presswalzen enthält, und einem Punkt, an dem der erste Führungskopf das erste elastische Element freigibt, 30 mm oder weniger beträgt.

2. Vorrichtung nach Anspruch 1, bei dem der erste Führungskopf das erste elastische Element zwischen die erste Bahn und eine zweite Bahn zuführt und
die beiden Presswalzen die erste Bahn, das erste elastische Element und die zweite Bahn zusammenpressen.

3. Vorrichtung nach Anspruch 1 oder 2, die ferner umfasst:
einen zweiten Bewegungsabschnitt, der in einer Richtung quer zu der ersten Bahn beweglich ist; und
einen zweiten Führungskopf, der an dem zweiten Bewegungsabschnitt ausgebildet ist, um ein zweites elastisches Element an eine Position, die sich stromaufseitig von der Position befindet, an der die erste Bahn gepresst wird, zuzuführen,
wobei wenigstens ein Teil des ersten Führungskopfes und wenigstens ein Teil des zweiten Führungskopfes gegenseitig überlappen, wenn der erste Führungskopf und der zweite Führungskopf auf eine Ebene, die zu den Achsen der beiden Presswalzen parallel ist, projiziert werden.

4. Vorrichtung nach einem der Ansprüche 1 bis 3, die ferner eine Steuereinheit umfasst, um die Bewegung des ersten Bewegungsabschnitts anhand von Musterinformationen, die ein Verlaufsmuster des auf der ersten Bahn angeordneten elastischen Elements angeben, von Bewegungsinformationen des ersten Bewegungsabschnitts und von Positionsinformationen der ersten Bahn zu steuern.

5. Vorrichtung nach Anspruch 1 oder 2, die umfasst:
einen zweiten Bewegungsabschnitt, der in einer Richtung quer zu der ersten Bahn beweglich ist;
einen zweiten Führungskopf, der an dem zweiten Bewegungsabschnitt ausgebildet ist, um ein zweites elastisches Element an eine Position stromaufseitig von der Position, an der die erste Bahn gepresst wird, zuzuführen; und
eine Steuereinheit, um die Bewegung des ersten Bewegungsabschnitts und des zweiten Bewegungsabschnitts anhand von Musterinformationen, die ein Verlaufsmuster der auf der ersten Bahn angeordneten elastischen Elemente angeben, und/oder von Bewegungsinformationen des ersten Bewegungsabschnitts und/oder von Bewegungsinformationen des zweiten Bewegungsabschnitts und/oder von Positionsinformationen der ersten Bahn und/oder von Positionsinformationen einer zweiten Bahn zu steuern.

6. Verfahren für die Herstellung von Bekleidungsartikeln, das die folgenden Schritte umfasst:
kontinuierliches oder intermittierendes Aufbringen eines Bindemittels auf wenigstens eine von zwei Bahnen;
Hin- und Herbewegen eines Führungskopfes, der wenigstens ein Durchgangsloch besitzt und einen Antriebsabschnitt aufweist, in einer Richtung quer zu wenigstens einer der beiden Bahnen, während ein elastisches Element durch das Durchgangsloch zwischen die beiden Bahnen zugeführt wird;
Steuern des Antriebsabschnitts anhand von Musterinformationen, die ein Muster des auf der Bahn angeordneten elastischen Elements angeben, und/oder von Bewegungsinformationen des Antriebsabschnitts und/oder von Positionsinformationen wenigstens einer der zwei Bahnen;
Pressen der beiden Bahnen und des dazwischen befindlichen elastischen Elements zwischen zwei Presswalzen, um eine komplexe Bahn zu bilden, wobei wenigstens eine der beiden Presswalzen einen Radius im Bereich von 15 mm bis 35 mm hat;
Bilden von Beinöffnungen durch die beiden Bahnen oder die komplexe Bahn;
Anordnen von Absorptionsmitteln auf der komplexen Bahn; und
Schneiden der komplexen Bahn mit den darauf angeordneten Absorptionsmitteln,
wobei in dem Schritt des Hin- und Herbewegens des Führungskopfes der Führungskopf hin und her bewegt wird und dabei ein Abstand zwischen einem Punkt, an dem der Führungskopf das elastische Element freigibt, und einer Ebene, die die Achsen der Presswalzen enthält, auf einem Wert von 30 mm oder weniger aufrechterhalten wird.

7. Verfahren nach Anspruch 6, das ferner die folgenden Schritte umfasst:
Falten der komplexen Bahn mit den durch sie gebildeten Öffnungen und den darauf angeordneten Absorptionsmitteln in der Weise, dass die Kanten der komplexen Bahn aufeinander ausgerichtet sind; und
Unterteilen der gefalteten komplexen Bahn mit Abdichtung, bevor die komplexe Bahn geschnitten wird.

8. Verfahren nach Anspruch 6, das ferner die folgenden Schritte umfasst:
Falten geschnittener Teile der komplexen Bahn, derart, dass die geschnittenen Kanten der komplexen Bahn aufeinander ausgerichtet sind; und
Abdichten wenigstens zweier Abschnitte jedes der gefalteten geschnittenen Teile der komplexen Bahn, so dass das gefaltete geschnittene Teil wenigstens drei Öffnungen besitzt.

## Revendications

1. Appareil de fabrication d'articles d'habillement, qui comprend :
deux rouleaux de pinçage pour pincer une première nappe et un premier élément élastique,
une première partie mobile qui peut être déplacée dans une direction qui croise la première nappe et
une première tête de guidage formée sur la première partie mobile et qui amène le premier élément élastique en une position située en amont d'une position dans laquelle la première nappe est pincée,
le rayon d'au moins l'un des rouleaux de la paire de rouleaux de pinçage étant d'environ 15 mm à environ 35 mm et
la distance D entre le plan qui contient les axes des deux rouleaux de pinçage et le point en lequel la première tête de guidage libère le premier élément élastique est d'environ 30 mm ou moins.

2. Appareil selon la revendication 1, dans lequel la première tête de guidage amène le premier élément élastique entre la première nappe et une deuxième nappe et dans lequel les deux rouleaux de pinçage pincent la première nappe, le premier élément élastique et la deuxième nappe.

3. Appareil selon les revendications 1 ou 2, qui comprend en outre :
une deuxième partie mobile qui peut être déplacée dans une direction qui croise la première nappe et
une deuxième tête de guidage formée sur la deuxième partie mobile et qui amène un deuxième élément élastique en une position située en amont de la position dans laquelle la première nappe est pincée,
au moins une partie de la première tête de guidage et au moins une partie de la deuxième tête de guidage se superposant mutuellement dans une projection de la première tête de guidage et de la deuxième tête de guidage dans un plan parallèle aux axes des deux rouleaux de pinçage.

4. Appareil selon l'une quelconque des revendications 1 à 3, qui comprend en outre un contrôleur qui contrôle le déplacement de la première partie mobile sur base d'informations de motif qui indiquent un motif de trace de l'élément élastique placé sur la première nappe, d'informations de déplacement de la première partie mobile et d'informations de position de la première nappe.

5. Appareil selon les revendications 1 ou 2, qui comprend en outre :
une deuxième partie mobile qui peut être déplacée dans une direction transversale par rapport à la première nappe,
une deuxième tête de guidage formée sur la deuxième partie mobile pour amener un deuxième élément élastique en une position située en amont de la position dans laquelle la première nappe est pincée et
un contrôleur qui contrôle le déplacement de la première partie mobile et de la deuxième partie mobile à partir d'au moins une information de motif qui indique un motif de trace des éléments élastiques placés sur la première nappe, d'informations de déplacement de la première partie mobile, d'informations de déplacement de la deuxième partie mobile, d'informations de position de la première nappe et d'informations de position d'une deuxième nappe.

6. Procédé de fabrication d'articles d'habillement, qui comprend les étapes qui consistent à :
appliquer en continu ou par intermittence un liant sur au moins l'une parmi deux nappes,
à l'aide d'une partie d'alimentation en énergie, déplacer en va-et-vient une tête de guidage traversée au moins par un trou, dans une direction qui croise au moins l'une des deux nappes tout en amenant un élément élastique entre les deux nappes par l'intermédiaire de la perforation,
contrôler la partie d'alimentation en énergie sur base d'au moins une information de motif qui indique un motif de l'élément élastique placé sur la nappe, d'informations de déplacement de la partie d'alimentation en énergie et d'informations de position d'au moins l'une des deux nappes,
pincer les deux nappes avec l'élément élastique placé entre elles entre deux rouleaux de pinçage pour former une nappe complexe, au moins l'un des deux rouleaux de pinçage ayant un rayon d'environ 15 mm à environ 35 mm,
former des ouvertures allongées à travers les deux nappes ou à travers la nappe complexe,
placer des agents d'absorption sur la nappe complexe et
découper la nappe complexe une fois que les agents d'absorption y ont été placés,
tandis que dans l'étape de déplacement en va-et-vient de la tête de guidage, la tête de guidage est déplacée en va-et-vient en maintenant, entre un point en lequel la tête de guidage libère l'élément élastique et le plan qui contient les axes des rouleaux de pinçage, une distance d'environ 30 mm ou moins.

7. Procédé selon la revendication 6, qui comprend en outre les étapes qui consistent à :
plier la nappe complexe dans laquelle les ouvertures ont été formées et sur laquelle les agents d'absorption ont été placés de telle sorte que les bords de la nappe complexe soient alignés l'un sur l'autre et
découper la nappe complexe repliée en la scellant avant de découper la nappe complexe.

8. Appareil selon la revendication 6, qui comprend en outre les étapes qui consistent à :
replier les pièces découpées de la nappe complexe de telle sorte que les bords découpés de la nappe complexe soient alignés l'un sur l'autre et
sceller au moins deux parties de chacune des pièces découpées et repliées de la nappe complexe de telle sorte que la pièce découpée et repliée présente au moins trois ouvertures.
